Europäisches Patentamt

European Patent Office

Office européen des brevets

(11) Publication number: **0 069 389**
**B1**

## (12) EUROPEAN PATENT SPECIFICATION

(45) Date of publication of patent specification: **12.11.86**

(21) Application number: **82106032.4**

(22) Date of filing: **06.07.82**

(51) Int. Cl.⁴: **A 61 B 17/36**

(54) Manipulator for laser knife.

(30) Priority: **07.07.81 JP 105077/81**

(43) Date of publication of application:
**12.01.83 Bulletin 83/02**

(45) Publication of the grant of the patent:
**12.11.86 Bulletin 86/46**

(84) Designated Contracting States:
**DE FR GB SE**

(56) References cited:
**GB-A-1 153 843**
**GB-A-1 429 843**
**US-A-3 821 510**
**US-A-3 914 015**
**US-A-4 027 938**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Takenaka, Shinya c/o Sumitomo Electr Industr Ltd**
**Osaka Works, No. 1-3 Shimaya 1-chome Konohana-ku, Osaka-shi, Osaka (JP)**
Inventor: **Katsuyoshi, Sunago c/o Sumitomo Electr Industr Ltd**
**Osaka Works, No.1-3 Shimaya 1-chome Konohana-ku, Osaka-shi, Osaka (JP)**

(74) Representative: **Henkel, Feiler, Hänzel & Partner**
**Möhlstrasse 37**
**D-8000 München 80 (DE)**

Courier Press, Leamington Spa, England.

# Description

## Background of the Invention
### *Field of the Invention*

This invention relates to a manipulator for a laser knife using an optical fiber light guide as a light conducting path, comprising: an elongated inflexible hand piece detachably coupled to a forward portion of said optical fibre light guide, said hand piece being shaped to enable the part to be irradiated by a laser to be confronted with a first end portion of said hand piece.

## Description of the Prior Art

A laser beam is monochromatic and excellent in directivity. When the laser beam is focused at a minute point, then great energy can be otained there. Therefore, the laser beam is utilized as a laser knife in the field of medical treatment, and especially when a small limited part of a body is subjected to medical treatment or surgical operation, the use of the laser knife is effective.

With the laser knife, a laser beam from a laser oscillating section is applied to the part of the body which is subjected to medical treatment or surgical operation. In this operation, it is required that the laser beam is applied correctly to an extremely small target. In order to meet this requirement, an optical fiber low in weight and excellent in flexibility is frequently used as the light conducting path.

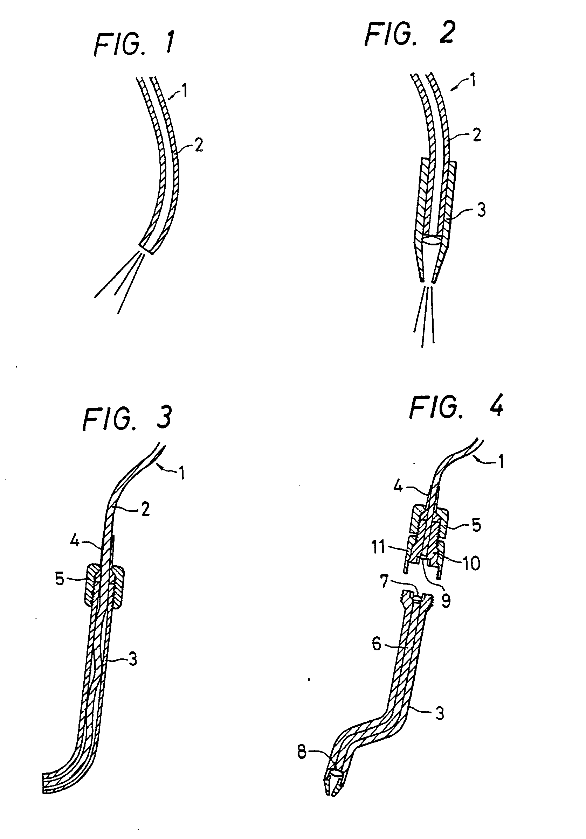

In most of the laser knife manipulators using the optical fibers as the light conducting paths, the laser beam is applied to the part to be irradiated, with the cover 2 of the optical fiber 1 directly gripped, as shown in Fig. 1, which is an explanatory diagram of the front end portion of the manipulator. Accordingly, the optical fiber 1 is liable to bend during irradiation, as a result of which it is impossible to correctly apply the laser beam to the part to be irradiated, and the temperature is increased by the reflected light.

In order to overcome these drawbacks, a laser knife manipulator has been proposed in the art, in which, as shown in Fig. 2, the front end portion of an optical fiber 1 is inserted into a hand piece 3 which is made of a straight pipe of metal or synthetic resin. However, the conventional manipulator still suffers from the drawback that it is difficult to apply the laser beam in the directions other than the axial direction of the hand piece 3; i.e., some parts cannot be irradiated with the laser beam, with the result that the range of application to medical treatment or surgical operation is limited.

A manipulator of the kind initially mentioned is disclosed essentially in US—A—3 821 510. There is described a laser beam transmitting a focusing device for application in medical and dental surgery. As described there, the known hand-held laser instrumentation device has a flexible conduit provided at its proximal end with three inlet connectors, each having screw threads formed externally thereon for connection to externally provided intakes. This hand-held device is not disposed over an optical fiber light guide, but one of the connectors is adapted for connection to an external source of laser radiation. A second connector attaches to a source of pressurized fluid such as an inert gas, while the third connector is attached to a source of gas for a medical or dental use.

Further, in the known manipulator, the distal end of the flexible conduit is provided with a laser instrumentation device or stylus attached to the flexible conduit with an annular coupling. The annular coupling provides therein with apertures for the passage of the fluid from a first chamber into a second chamber formed within the tubular section of the stylus. Thus, the hand-held laser instrumentation device of the reference is quite complicated in design; and it would be complicated to change different hand pieces.

## Summary of the Invention

Accordingly, it is an object of the present invention to provide a manipulator as initially defined, in which a number of hand-pieces which have been shaped as desired according to the configurations of the part to be irradiated with the laser beam can be selectively and readily used. This includes that the change of hand pieces can be executed in a simple manner.

This object is achieved in accordance with a first concept of the present invention in that said manipulator comprises further an elastic sleeve member having an O-ring shaped protrusion at one of its ends which is positioned adjacent a second end portion of said hand piece, and being held to said fiber light guide under its own elastic force, and a connector cap having female threads on the inner wall thereof, the cap being disposed over said optical fiber light guide and said elastic sleeve member and adapted to couple said hand piece to said fiber light guide by screwing the connector cap on a threaded portion of the second end of said hand piece.

The object is also achieved in accordance with a second concept of the present invention in that said manipulator comprises further an elastic sleeve member being positioned over the forward portion of said fiber light guide and held to said fiber light guide under its own elastic force, said elastic sleeve member having an O-ring shaped protrusion at one of its ends which is positioned near the forward end of said fiber light guide, a holder being positioned over the forward end portion of said fiber light guide and having male threads on the outer wall thereof, a connector cap having female threads on the inner wall thereof, the cap being disposed over said optical fiber light guide and said elastic sleeve member and threadably engaged with a threaded portion of said holder in order to couple said holder to said O-ring shaped protrusion of said elastic sleeve member and a connector coupler being disposed over said holder and the forward end of said optical fiber light guide and being adapted to fixedly couple the second end of said hand piece to said holder and to optically couple an optical fiber provided in said hand piece to said optical fiber light guide.

The characteristic features of the invention will become more apparent from the following detailed description when read in conjunction with the accompanying drawings in which like parts are designated by like reference numerals.

Brief Description of the Drawings

In the accompanying drawings:

FIG. 1 is a longitudinal sectional view of the emergence end portion of an example of a conventional laser knife manipulator;

FIG. 2 is a longitudinal sectional view of the emergence end portion of another example of a conventional laser knife manipulator;

FIG. 3 is a longitudinal sectional view of the emergence end portion of an example of a laser knife manipulator according to this invention; and

FIG. 4 is a longitudinal sectional view of the emergence end portion of another example of the laser knife manipulator according to the invention.

Detailed Description of the Preferred Embodiments

A first example of a laser knife manipulator according to this invention as shown in Fig. 3, comprises: an optical fiber 1 as a light conducting path adapted to conduct a laser beam from a laser oscillating section (not shown); and a hand piece 3 detachably put over the end portion of the optical fiber 1. The hand piece 3 is suitably shaped according to the configuration and the position of the part to be irradiated with the laser beam. More specifically, the hand piece 3 is made of metal or synthetic resin and has an inside diameter which is slightly larger than the outside diameter of the cover 2 of the optical fiber. One end portion of the hand piece 3 is bent as shown in Fig. 3. Male threads are formed on the outer wall of the other end portion of the hand piece 3. The manipulator further comprises: a rubber sleeve 4 having an O-ring-shaped protrusion at one end; and a cap 5 having female threads on the inner wall thereof. The rubber sleeve 4 is put over the optical fiber's cover 2 in such a manner that the rubber sleeve 4 is set beside the hand piece 3 and is held there by the elastic force of itself. Under this condition, the cap 5 is screwed onto the threaded portion of the hand piece.

In other words, first the cap 5 and the rubber sleeve 4 are put over the optical fiber's cover 2, and then the optical fiber 1 is inserted into the hand piece 3. Under this condition, the cap 5 is screwed on the threaded portion of the hand piece 3. As a result, the O-ring-shaped protrusion of the rubber sleeve 4 is pressed against the hand piece 3, so that the hand piece 3 is fixedly secured to the cover 2.

It goes without saying that the hand piece 3 can be readily removed from the optical fiber 1 by unscrewing the cap 5.

A variety of hand pieces such as straight hand pieces and bent hand pieces different in the radius of curvature can be fabricated separately according to the configurations and the positions of the parts to be irradiated. Accordingly, with a suitable one of these hand pieces, the laser beam can be applied correctly to the armed part, and the part to be irradiated will never be covered by the operator's hand.

If the hand piece 3, the rubber sleeve 4 and the cap 5 are removed from the optical fiber 1, then the latter can be readily inserted into the laser knife inserting hole of a conventional endoscope, so that it can be used with the endoscope.

Another example of the laser knife manipulator according to the invention is as shown in Fig. 4. In this example, an optical fiber 6 is fixedly inserted into a hand piece 3 which has been shaped as desired, and when the hand piece 3 is secured to an optical fiber 1, the latter 1 is optically coupled to the optical fiber 6.

For this purpose, a coupling lens 7 is fixedly secured to the coupling end of the optical fiber 6 in the hand piece 3. In this example, a lens 8 for condensing the laser beam is fixed to the other end of the optical fiber 6 in the hand piece 3. Similarly as in the first example shown in Fig. 3, a rubber sleeve 4 and a cap 5 are put over the optical fiber 1 which is coupled to the hand piece 3. A lens holder 10 holds a coupling lens 9 which is set at the front end of the optical fiber 1. The lens holder 10 is threadably engaged with the cap 5. A coupling nut 11 is provided on the lens holder 10. The coupling nut 11 is used to fixedly couple the lens holder 10 to the hand piece 3.

First, the front end portion of the optical fiber 1, on which the rubber sleeve 4 and the cap 5 have been mounted, is inserted into the lens holder 10. Then, the cap 5 is screwed on the lens holder 10, so that the latter 10 is fixedly secured to the front end portion of the optical fiber. Then, the coupling nut 11 is tightened so that the hand piece 3 with the optical fiber 6 is fixedly secured to the lens holder 10. As a result, the optical fiber 1 is optically coupled to the optical fiber 6, and the hand piece 3 is fixedly secured.

In the second example described above, two lenses are used for optical coupling; however, the number of lenses may be one or more than two.

As is apparent from the above description, a number of hand pieces which have been shaped as desired according to the configurations of the part to be irradiated with the laser beam can be selectively and readily used. Therefore, the laser beam can be applied correctly to the part to be irradiated. Furthermore, the invention makes it possible for one laser knife to perform the dental surgical operation, the oral surgical operation, the surgical operation, and so on. In addition, the laser knife manipulator can be readily coupled to an endoscope. Thus, the laser knife manipulator according to the invention has a wide range of application.

**Claims**

1. A manipulator for a laser knife using an optical fiber light guide as a light conducting path, comprising:

an elongated inflexible hand piece (3) detachably coupled to a forward portion of said optical fiber light guide (1), said hand piece (3) being shaped to enable the part to be irradiated by a laser to be confronted with a first end portion of said hand piece (3), characterized in further comprising:

an elastic sleeve member (4) having an O-ring shaped protrusion at one of its ends which is positioned adjacent a second end portion of said hand piece (3), and being held to said fiber light guide (1) under its own elastic force, and a connector cap (5) having female threads on the inner wall thereof, the cap (5) being disposed over said optical fiber light guide (1) and said elastic sleeve member (4) and adapted to couple said hand piece (3) to said fiber light guide (1) by screwing the connector cap (5) on a threaded portion of the second end of said hand piece (3).

2. A manipulator for a laser knife using an optical fiber light guide as a light conducting path, comprising:

an elongated inflexible hand piece (3) detachably coupled to a forward portion of said optical fiber light guide (1), said hand piece (3) being shaped to enable the part to be irradiated by a laser to be confronted with a first end portion of said hand piece (3), characterized in further comprising:

an elastic sleeve member (4) being positioned over the forward portion of said fiber light guide (1) and held to said fiber light guide (1) under its own elastic force,

said elastic sleeve member (4) having an O-ring shaped protrusion at one of its ends which is positioned near the forward end of said fiber light guide (1), a holder (10) being positioned over the forward end portion of said fiber light guide (1) and having male threads on the outer wall thereof, a connector cap (5) having female threads on the inner wall thereof, the cap (5) being disposed over said optical fiber light guide (1) and said elastic sleeve member (4) and threadably engaged with a threaded portion of said holder (10) in order to couple said holder (10) to said O-ring shaped protrusion of said elastic sleeve member, and a connector coupler (11) being disposed over said holder (10) and the forward end of said optical fiber light guide (1) and being adapted to fixedly couple the second end of said hand piece (3) to said holder (10) and to optically couple an optical fiber (6) provided in said hand piece (3) to said optical fiber light guide (1).

## Revendications

1. Manipulateur de scalpel à laser, mettant en oeuvre un guide lumineux à fibre optique comme trajet conducteur de la lumière, comprenant une pièce à main (3) allongée et non souple, couplée de manière amovible à une partie antérieure du guide lumineux (1) à fibre optique, la pièce à main (3) ayant une configuration telle que la partie à irradier par un laser peut être placée en face de la première extrémité de la pièce à main (3), caractérisé en ce qu'il comprend en outre un organe (4) formant un manchon élastique ayant une saillie en forme de joint torique à une première extrémité, placé près d'une seconde extrémité de la pièce à main (3) et maintenu sur le guide lumineux (1) sous l'action de sa propre force d'élasticité, et un capuchon (5) de raccordement ayant un taraudage à sa paroi interne, le capuchon (5) étant placé sur le guide lumineux (1) à fibre optique et sur l'organe (4) formant un manchon élastique, et étant destiné à coupler la pièce à main (3) au guide lumineux (1) par vissage du capuchon (5) de raccordement sur une partie filetée de la seconde extrémité de la pièce à main (3).

2. Manipulateur de scalpel à laser mettant en oeuvre un guide lumineux à fibre optique comme trajet conducteur de lumière, comprenant une pièce à main allongée (3) qui n'est pas souple et qui est couplée de façon amovible à une partie antérieure du guide lumineux (1) à fibre optique, la pièce à main (3) ayant une configuration qui permet la mise de la partie à irradier par un laser en face d'une première partie d'extrémité de la pièce à main (3), caractérisé en ce qu'il comprend en outre un organe (4) sous forme d'un manchon élastique, placé sur la partie antérieure du guide lumineux (1) à fibre et maintenu sur ce guide lumineux (1) par sa propre force d'élasticité, l'organe (4) formant un manchon élastique ayant une saillie en forme de joint torique à une première extrémité, placée près de l'extrémité antérieure du guide lumineux (1),

un support (10) placé sur la partie antérieure du guide lumineux (1) et ayant un filetage à sa paroi externe,

un capuchon (5) de raccordement ayant un taraudage à sa paroi interne, le capuchon (5) étant placé sur le guide lumineux (1) à fibre optique et sur l'organe (4) formant un manchon élastique et étant vissé sur une partie filetée du support (10) afin que le support (10) soit couplé à la saillie en forme de joint torique de l'organe en forme de manchon élastique, et

un accouplement (11) de raccordement placé sur le support (10) et sur l'extrémité avant du guide lumineux (1) et destiné à coupler à demeure la seconde extrémité de la pièce à main (3) et le support (10) et à coupler optiquement une fibre optique (6) placée dans la pièce à main (3) au guide lumineux (1) à fibre optique.

## Patentansprüche

1. Einrichtung zum Manipulieren eines Lasermessers unter Verwendung eines Lichtleitfaserstrangs als Lichtleitstrecke, umfassend ein längliches, unbiegsames (starres) Griffstück (3), das trennbar mit einem vorderen Abschnitt des Lichtleitfaserstrangs (1) verbunden und so geformt ist, daß der mittels eines Lasers zu bestrahlende Teil zu einem ersten Endabschnitt des Griffstücks (3) in Gegenüberstellung bringbar ist, gekennzeichnet durch ein elastisches Hülsenelement (4), das einen o-ringförmigen Fortsatz an seinem einen Ende, das neben einem zweiten Endabschnitt des Griffstücks (3) angeordnet ist, aufweist und das unter seiner eigenen Elastizitätskraft am Lichtleitfaserstrang (1) gehalten wird, und eine an ihrer Innenfläche mit einem Innengewinde versehene Anschlußkappe (5), die über den Lichtleitfaserstrang (1) und das elastische Hülsenelement (4) aufgesetzt ist und das Griffstück (3) durch Aufschrauben der Anschlußkappe (5) auf einen Gewindeabschnitt am zweiten Ende des Griffstücks (3) mit dem Lichtleitfaserstrang (1) zu verbinden vermag.

2. Einrichtung zum Manipulieren eines Lasermessers unter Verwendung eines Lichtleitfaserstrangs als Lichtleitstrecke, umfassend ein längliches, unbiegsames (starres) Griffstück (3), das trennbar mit einem vorderen Abschnitt des Lichtleitfaserstrangs (1) verbunden und so geformt ist, daß der mittels eines Lasers zu bestrahlende Teil zu einem ersten Endabschnitt des Griffstücks (3) in Gegenüberstellung bringbar ist, gekennzeichnet durch ein elastisches Hülsenelement (4), das auf den vorderen Abschnitt des Lichtleitfaserstrangs (1) aufgesetzt ist und unter seiner eigenen Elastizitätskraft am Lichtleitfaserstrang (1) gehalten wird, wobei das elastische Hülsenelement (4) einen o-ringförmigen Fortsatz an seinem einen Ende aufweist, das nahe dem vorderen Ende des Lichtleitfaserstrangs (1) angeordnet ist,

einen auf den Vorderendabschnitt des Lichtleitfaserstrangs (1) aufgesetzten Halter (10) mit einem Außengewinde an seiner Außenfläche,

eine an ihrer Innenfläche mit einem Innengewinde versehene Anschlußkappe (5), die über den Lichtleitfaserstrang (1) und das elastische Hülsenelement (4) aufgesetzt und mit einem Gewindeabschnitt des Halters (10) verschraubt ist, um den Halter (10) mit dem o-ringförmigen Fortsatz des elastischen Hülsenelements zu verbinden, und

eine auf den Halter (10) und das Vorderende des Lichtleitfaserstrangs (1) aufgesetzte Anschlußkupplung (11) zum festen Verbinden des zweiten Endes des Griffstücks (3) mit dem Halter (10) und zum optischen Koppeln einer im Griffstück (3) vorgesehenen optischen Faser (6) mit dem Lichtleitfaserstrang (1).

*FIG. 1*

*FIG. 2*

*FIG. 3*

*FIG. 4*